# EUROPEAN PATENT APPLICATION

(11) **EP 1 252 818 A1**
(43) Date of publication of application: **30.10.2002**
(21) Application number: 00985829.1
(22) Date of filing: 21.12.2000
(51) Int. Cl.: A01K 67/027, C12N 5/18, C12N 15/12

(54) **METHOD OF TRANSFERRING MUTANT MITOCHONDRIAL DNA INTO GENITAL CELLS**

(30) Priority: 18.01.2000 JP 2000009194
(71) Applicant: Institute of Tsukuba Liaison Co., Ltd, Tsukuba-shi, Ibaraki 300-2642 (JP)
(72) Inventor: HAYASHI, Jun-Ichi, Abiko-shi, Chiba 270-1152 (JP)
(74) Representative: Woods, Geoffrey Corlett
(86) International application number: JP0009111
(87) International publication number: WO01052642

(57) **Abstract**

Provided is an effective method for introducing mutant mtDNA into germ cells, which comprises fusing enucleated cells possessing mutant mitochondrial DNA with mitochondrial DNA-less cells to produce cybrids, enucleating the cybrids, and fusing the cybrids with germ cells.

## Description

### TECHNICAL FIELD

The present invention relates to a method for introducing mutant mitochondrial DNA into a germ cell, and a mitochondrial DNA-knockout mouse which is produced by the method.

### BACKGROUND ART

Mutant mitochondrial DNAs have been implicated in mitochondrial diseases, aging, and a variety of age-related disorders (Wallace, D. C. Mitochondrial diseases in man and mouse. Science 283, p1482-1488 (1999); Larsson, N.-G. & Clayton, D. A. Molecular genetic aspects of human mitochondrial disorders. Annu. Rev. Genet. 29, p151-178 (1995). However, there is no convincing evidence to explain the pathogenesis.

Generation of mitochondrial DNA (hereinafter referred to as "mtDNA") -knockout mice can provide an ideal experimental system for elucidating how mutant mtDNAs are transmitted and distributed in tissues resulting in pathogenesis of mitochondrial diseases expressing various clinical phenotypes. Currently, no effective method for introducing mutagenized whole mtDNAs into the mitochondria of another cell has been developed, and all previous efforts to produce the above knockout mice have failed.

### DISCLOSURE OF THE INVENTION

The present invention has been achieved based on the above-mentioned technical background, and the purpose of the present invention is to provide a method to introduce mutant mtDNA into the mitochondria of germ cells.

As a result of thorough studies to solve the above problems, we have completed the present invention based on the findings that mitochondria carrying mutant mtDNA can be introduced successfully into germ cells by fusing an enucleated cell carrying mutant mtDNA with a mtDNA-less cell to produce a cybrid, enucleating the cybrid, and then fusing the enucleated cybrid with a germ cell.

In particular, the present invention provides a method for introducing mutant mtDNA into a germ cell, which comprises enucleating a cell carrying mutant mtDNA, fusing the enucleated cell with a mtDNA-less cell to produce a cybrid, enucleating the cybrid, and then fusing the enucleated cybrid with a germ cell.

Detailed description of the present invention is given as follows.

The method of the present invention for introducing mutant mtDNA into a germ cell comprises enucleating a cell carrying mutant mtDNA, fusing the enucleated cell with a mtDNA-less cell to produce a cybrid, enucleating the cybrid, and then fusing the enucleated cybrid with a germ cell.

An example of a cell carrying mutant mtDNA that can be used herein is, but is not limited to, a cell carrying deletion mutant mtDNA4696.

We have established a cell carrying the deletion mutant mtDNA4696.

Cells carrying mutant mtDNA can be enucleated by ,for example, cytochalasin B treatment and high-speed centrifugation as described in Hayashi, J-I. et al., J. Biol. Chem 269, 19060-19066 (1994).

Examples of mouse mtDNA-less cells, that is, mouse ρ⁰ cells that can be used herein include, but are not limited to, ρ⁰ C2C12 cells. We have established the mouse ρ⁰ cells (for detail, see Inoue, K. et al., Isolation of mitochondrial DNA-less mouse cell lines and their application for trapping mouse synaptosomal mitochondrial DNA with deletion mutations. J. Biol. Chem. 272, 15510-15515; Inoue, K. et al., Isolation and characterization of mitochondrial DNA-less lines from various mammalian cell lines by application of an anticancer drug, ditercalinium. Biochem. Biophys. Res. Commun. 239, 257-260(1997)).

Enucleated cells carrying mutant mtDNA can be fused with mtDNA-less cells by, for example, polyethylene glycol treatment as described in Hayashi, J-I. et al., J. Biol. Chem. 269, 19060-19066 (1944).

Cybrids produced from the above fusion procedure can be enucleated in a manner similar to enucleation of cells carrying mutant mtDNA.

As germ cells, for example, pro-nuclear stage embryos can be used, but are not limited thereto.

Cybrids and pro-nuclear stage embryos are fused by electrofusion.

Using germ cells produced as described above into which mutant mtDNAs have been introduced, mice can be produced.

In this case, embryos with the mtDNAs introduced therein are cultured upto the 4-cell stage in culture, and then the embryos are transplanted into the oviducts of female mice (pseudopregnant), thereby generating F0 mice.

Since mtDNA is always transmitted maternally, progeny mice can be generated by mating these F0 female mice with male mice having normal mtDNAs.

This application claims a priority from Japanese Patent Application No. 2000-9194, the disclosure of which is incorporated herein by reference.

### BRIEF DESCRIPTION OF THE DRAWINGS

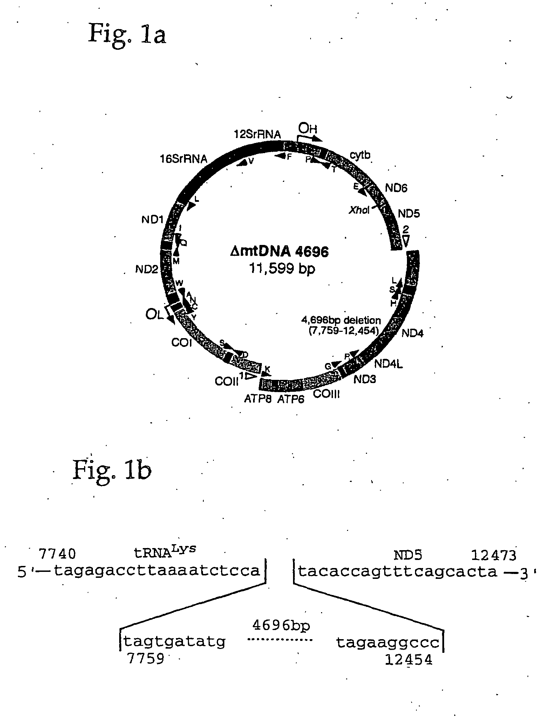

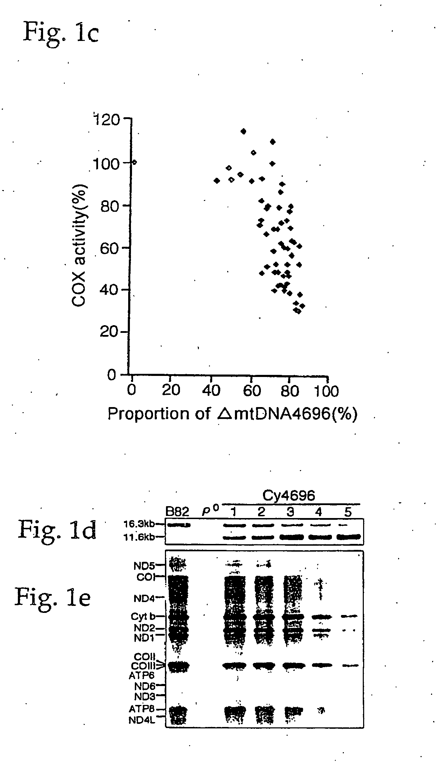

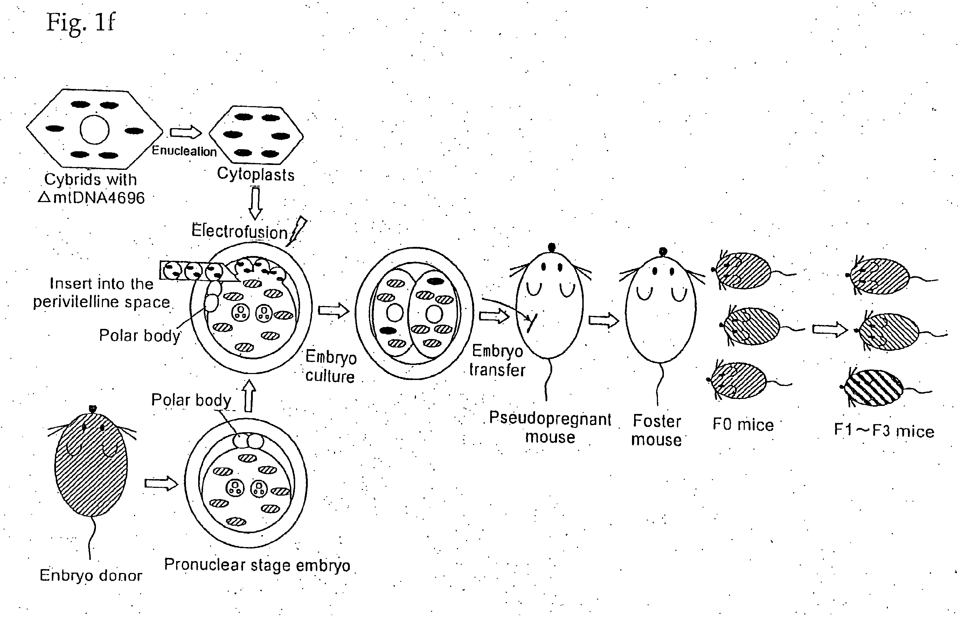

Figure 1 shows the genetic characterization of ΔmtDNA4696 in somatic cells and its application for generating mtDNA-knockout mice.
   1a: Gene map of ΔmtDNA4696. A region located outside the circle is deleted from the normal mtDNA. White arrows 1 and 2 indicate primer pairs used for sequencing and detecting ΔmtDNA4696.
   1b: The nucleotide sequence fo light strand mtDNA around the region of the deletion breakpoint of ΔmtDNA4696. A 4696bp sequence (the lower sequence in Fig. 1) comprising nucleotides 7759-12454 is a deleted,region.
   1c: Correlation between the proportion (%) of ΔmtDNA4696 and COX activity.
   1d: Cy4696 fragment (obtained by digestion with restriction enzymes) detected by the Southern Blotting. In the figure, bands at 16.3-kb and 11.6-kb indicate normal mtDNA and ΔmtDNA4696, respectively.
   1e: [³⁵S] methionine incorporation by the mitochondria of Cy4696 cybrid cell line. In the figure, ND5 to ND4L respectively correspond to polypeptides encoded by normal mtDNA.
   1f: Experimental scheme to generate mice having normal mtDNA and ΔmtDNA4696 derived from Cy4696 cybrid cell line in the heteroplasmic state. Mice and mitochondria having normal mtDNA are represented by " 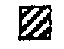 "; those having ΔmtDNA4696 by " ■ "; heteroplasmic mice having a small amount of ΔmtDNA4696 by " 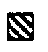 "; and mice having a large amount of ΔmtDNA4696 by " 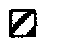 ".

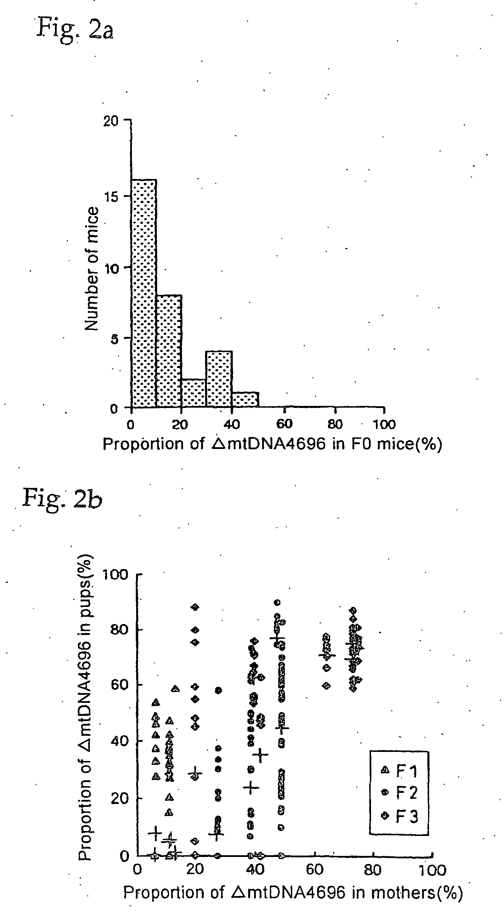

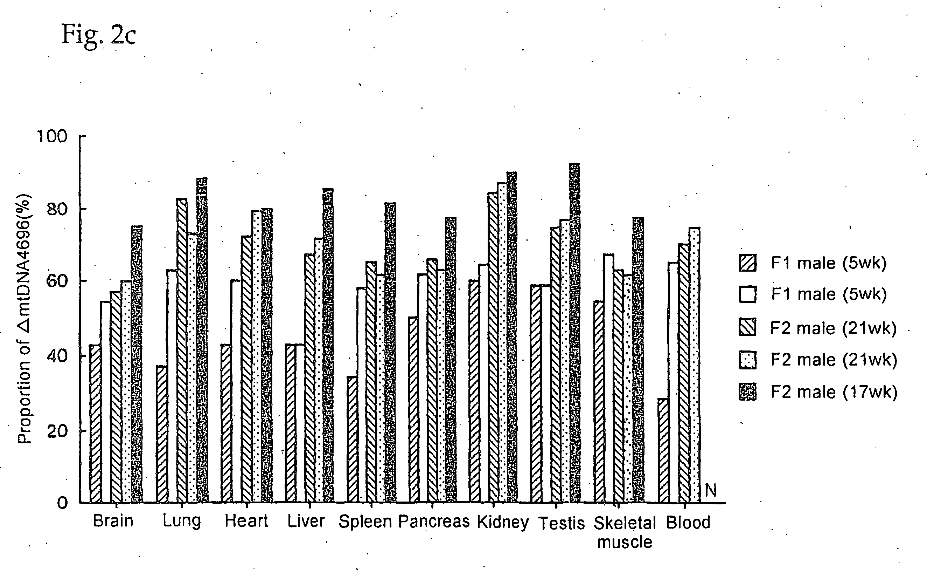

Figure 2 shows transmission and distribution of ΔmtDNA4696 in F0 to F3 mice.
   2a: The proportion of ΔmtDNA4696 in muscle biopsy from F0 mice is shown by histogram.
   2b: Comparison of the proportion of ΔmtDNA4696 transmitted from F0 to F3 mice made between mothers and their progeny mice. Female mice (five F0 mice, four F1 mice and seven F2 mice) were used as mothers for F1 (▲), F2 (●) and F3 (◆) progeny mice, respectively. Symbol "+" indicates average levels of ΔmtDNA4696.
   2c: ΔmtDNA4696 (proportion (%)) distributed in each tissue of a mouse.

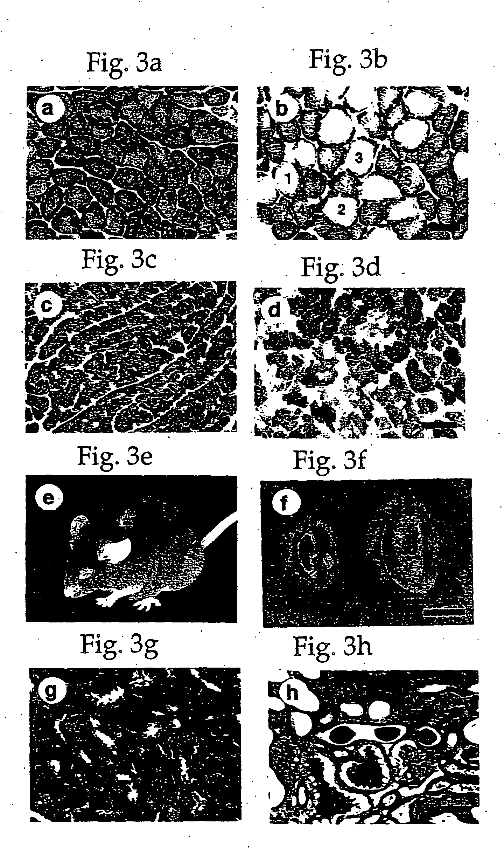

Figure 3 contains photographs showing histochemical and morphological abnormalities observed in normal mice and mice having ΔmtDNA4696.
   3a and 3b: Results of staining for COX activity of muscle fiber cross sections of skeletal muscle. Muscle biopsy sample is from a normal male mouse (3a), and from an F2 mouse having ΔmtDNA4696 (3b).
   3c and 3d: Results of staining for COX activity of muscle fiber in cross sections of cardiac muscle. Muscle biopsy sample is from a normal male mouse (3c), and from an F2 mouse having ΔmtDNA4696 (3d).
   3e: Characteristic appearance of an F1 male mouse.
   3f: Kidneys from a mouse having ΔmtDNA (right) and a normal mouse (left).
   3g and 3h: Renal cortex from a mouse having ΔmtDNA (3h) and a normal mouse (3g).

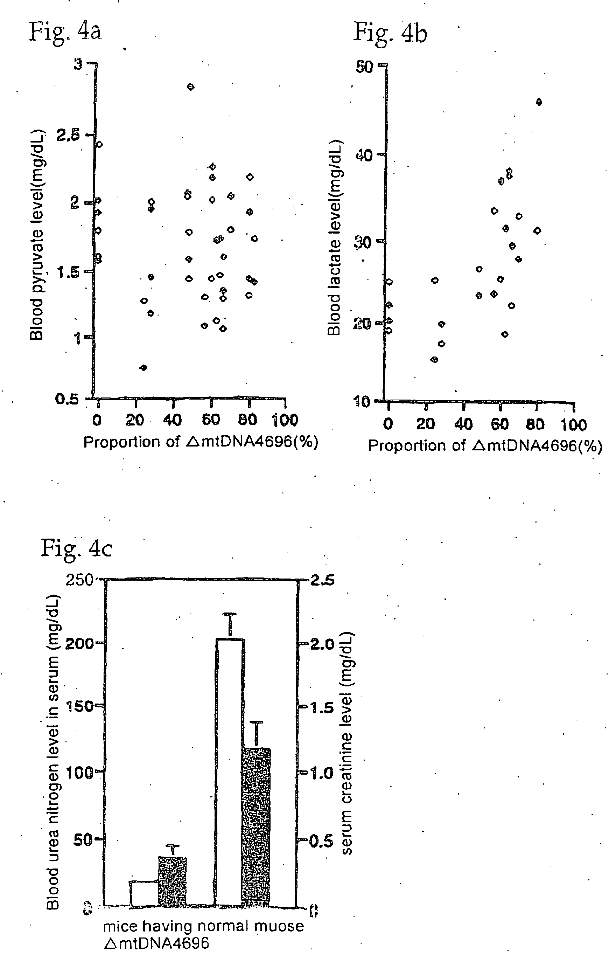

Figure 4 shows biochemical abnormalities in the blood of a mouse having ΔmtDNA4696.
   4a and 4b: Effect of the proportion of ΔmtDNA4696 on blood pyruvate (4a) and lactate (4b) levels. White and black diamond symbols correspond respectively to levels measured before and after glucose administration.
   4c: Comparison of serum urea nitrogen levels (white bar graph) and serum creatinine levels (black bar graph) between mice having normal mtDNA and mice having ΔmtDNA4696.

### BEST MODE FOR CARRYING OUT THE INVENTION

The present invention is further described in the following examples. The examples are provided for illustrative purposes only, and are not intended to limit the scope of the invention.

### Example 1

### Introduction of deletion mutant mtDNA into pro-nuclear stage embryos

### (1) Preparation of cybrids carrying deletion mutant mtDNA (hereinafter, referred to as "ΔmtDNA")

### (1.1) Introduction of ΔmtDNA into mtDNA-less cells (ρ⁰ cells)

Enucleated culture cells (NIH3T3 cells) were fused with mouse mtDNA-less cells (that is, ρ⁰ cells) by polyethylene glycol (PEG) treatment, thereby producing 26 cybrid clones.

### (1.2) Selection of cybrid carrying ΔmtDNA

To select cybrids into which ΔmtDNA had been successfully introduced, PCR was (PCR conditions: 30 cycles of 94 °C for 60 sec, 45 °C for 60 sec, and 45 °C for 120 sec) performed using the following primer set.

### (Primer set)

Forward primer: 5'-aagaaaggaaggaatcgaaccccct-3' (SEQ ID NO: 1: corresponding to the
nucleotide sequence 6871-6895 of normal mtDNA)

Reverse primer: 5'-tatgttggaaggagggattggggta-3' (SEQ ID NO: 2: corresponding to the
nucleotide sequence 13666-13642 of normal mtDNA)

Signal amplified by PCR was observed for 8 out of 26 cybrid clones, and then the amount of ΔmtDNA of these 8 clones was quantified by the Southern blotting.

The amounts of ΔmtDNA contained in all but one of these clones were not sufficient to be detected by the Southern blotting. Only one clone, Cy4696, possessed 30% ΔmtDNA. The mutation was derived from the mouse culture cells.

Nucleotide sequence analysis on PCR products revealed that ΔmtDNA carried by the cybrid clone Cy4696 (hereinafter, referred to as "ΔmtDNA4696) had 4696 bp deletion corresponding to nucleotide sequence 7759-12454 of normal mtDNA. The deleted region contained 6 tRNA genes arid 7 structural genes (Fig. 1a shows the gene map of ΔmtDNA4696). Unlike other mouse ΔmtDNA reported so far, the ΔmtDNA4696 contained no direct repeat around the region carrying the deletion (Fig. 1b shows the nucleotide sequence around the deleted region in the L strand of ΔmtDNA4696).

The proportion of ΔmtDNA 4696 in Cy4696 cybrid increased from 30% to 83% while culturing of the cells at 37 °C for 30 months, but increases exceeding this level have not been observed in longer period culturings. These behaviors were very similar to those observed for human cybrids in which ΔmtDNA that had been shown to be responsible for pathogenesis of a mitochondrial disease, Kearns-Sayre syndrome, had been introduced. Probably, the size of ΔmtDNA is small enough to be advantageous upon replication, so that it is predominant over the normal mtDNA during culturing for a long period of time.

### (2) Confirmation of pathogenicity of ΔmtDNA4696

Next, the pathogenicity of ΔmtDNA4696 was confirmed by the following procedures.

For cybrid Cy4696 clones carrying ΔmtDNA4696 with varied proportions (50% to 90%), respiratory activity (that is, cytochrome c oxidase (COX activity)) was measured as the proportion (%) of cyanide-sensitive oxidation by reduced cytochrome c according to the technique of Miyabayashi et al (Miyabayashi, S. et al., 1984, Brain Dev. 6, 362-372). Effect of the proportion of ΔmtDNA4696 on COX activity was analyzed. The results are shown in Figure 1c.

Further, five Cy4696 cybrid cell lines (containing 56%, 67%, 82%, 84% and 85% ΔmtDNA, respectively), mouse ρ⁰ cells and B82 cells (ρ⁰ parent cell line) were digested with restriction enzyme XhoI. The resulting fragments were detected by the Southern blotting. The results are shown in Figure 1d.

Furthermore, translation products within the mitochondria of each of the five Cy4696 cybrid cell lines, mouse ρ⁰ cells and B82 cells (same as those described above) were labeled by [³⁵S] methionine incorporation. Mitochondrial fractions were then isolated, and the patterns of SDS electrophoresis were analyzed with BAS2000, so as to assess the activity of the mitochondrial translation system. The results are shown in Figure 1e.

### (Result)

Comparison of Fig. 1c, 1d and 1e revealed that the cybrid with a high proportion of ΔmtDNA 4696 showed decreases in both COX activity and the activity of the mitochondrial translation system, proving the pathogenicity of ΔmtDNA4696.

Based on the fact that all the cybrids tested herein had the same nuclear background with that of ρ⁰ cells, the parent cell line, it is thought that a reduction in the activity of the entire mitochondrial translation system and the resulting reduction in COX activity were caused by the accumulation of ΔmtDNA4696 lacking 6 tRNA genes.

### (3) Introduction of Cy4696 cybrids into mouse pro-nuclear stage embryos

Cy4696 cybrids were enucleated by cytochalasin B treatment and high speed centrifuge, thereby producing several cytoplasts.

51U of pregnant mare serum gonadotrophin (PMSG) and 51U of human chorionic gonadotropin (hCG) were sequentially injected intraperitoneally at an interval of 48 hours to 300 hybrids F1 female mice (8- to 10-weeks-old, B6D2F1) to induce superovulation.

These female mice were caged together with fertile B6D2F1 male mice overnight for mating.

At 15 to 18 hours after injection with hCG, theoviducts of the female mice were punctured, and then pro-nuclear stage embryos were collected. The collected embryos were transferred into CZB media, and then allowed to stand in the CZB media containing hyaluronidase (SIGMA), thereby removing cumulus cells.

Using a piezo-driven micromanipulator, approximately 10 cytoplasts obtained as described above were injected into the perivitelline space of embryos whose cumulus cells had been removed.

Subsequently, pulse stimulation (3000 or 3500 V/cm, 10 µsec) was applied in the presence of alternating electric field (before and after fusion: 100 V/cm, 2 MHz, 30 sec) to fuse the cytoplasts with the embryos. (Note: The alternating electric field employed before and after fusion is for dielectrophoresis to keep the cells adhered to each other)

### (Result)

Most embryos (95% or more) survived after electrofusion. When pro-nuclear stage embryos fused with the cytoplasts derived from Cy4696 were cultured on petri dishes, 1142 embryos developed into 2-cell stage embryos after 24 hours of culturing, and to 4-cell stage embryos after 48 hours of culturing.

### Example 2

### Production of mtDNA-knockout mice

mtDNA-knockout mice were produced using embryos carrying ΔmtDNA4696 prepared in Example 1.

### (1) Production of F0 mice

First, 1142 4-cell stage embryos (possessing ΔmtDNA4696) obtained in Example 1 were transferred into the oviducts of ICR female mice (8- to 12-weeks-old, pseudopregnant female) on day 1 after pseudo-pregnancy treatment. Here, the term "on day 1 after treatment" refers to the next day after mating (pseudo-pregnancy) with vasoligated male mice.

On day 20 after pseudo-pregnancy treatment, the uterus of the pseudoparent female mouse was subjected to cesarean section for examination, and surviving progeny mice (F0 mice) were reared by ICR female mice (foster parent) capable of lactation. Figure 1f summarizes the series of procedures following procedure described in Example 1 (3) (introduction of Cy4696 cybrids into mouse pro-nuclear stage embryos).

98 out of 111 newborn mice from the pseudo-parent mice grew into adult. The presence of ΔmtDNA4696 was examined by the PCR method using DNA extracted from the tails of these mice. As a result, 31 out of 98 individual mice were shown to possess ΔmtDNA4696 in their somatic cells. A pair of primers and conditions employed for PCR are as follows.

### (Primer pair)

Forward primer : 5'-AACAGTAACATCAAACCGACCAGG-3' (SEQ ID NO: 3
corresponding to nucleotides 7558-7581 of the nucleotide sequence of normal mtDNA)

Reverse primer : 5'-CTATTATCAGGCCTAGTTGGC-3' (SEQ ID NO: 4 corresponding
to nucleotides 12678-12658 of the nucleotide sequence of normal mtDNA)

### (PCR conditions)

30 cycles, each cycle consisting of 94 °C for 1 min, 45 °C for 1 min and 72 °C for 1 min

Muscle biopsy was performed for the musculus tibialis anterior (M. tibialis anterior) of 31 F0 mice having ΔmtDNA4696, and then the proportion of ΔmtDNA4696 was measured by the Southern blotting. 24 mice were shown to have 5.7 to 41.8% ΔmtDNA4696 (The results are shown in Figure 2a).

### (2) Generation of progeny mice possessing ΔmtDNA4696

mtDNA inherits strictly maternally. Thus, 5 founder F0 females having 5.7%, 6.1%, 10.8%, 11.2% and 13.0% ΔmtDNA4696, respectively, were selected as mothers, and then allowed to mate with male mice (BDF1) to breed F1 mice.

Similarly, F2 mice from F1 female mice, and then F3 mice from F2 female mice were born.

### (3) Proportion of ΔmtDNA4696 transmitted through generations and distribution of ΔmtDNA4696 among tissues

The proportions of ΔmtDNA4696 transmitted from individual F0 to F3 mice (mothers and progeny mice) obtained as described in (2) were compared.

Comparisons of the proportions of ΔmtDNA4696 were carried out between F0 and F1 mice, F1 and F2 mice, and F2 and F3 mice, using muscle biopsy samples of tibialis anterior. Five F0 female mice, four F1 female mice and seven F2 female mice were used respectively as mother mice for producing the next generations. The five F0 mother mice had 5.7%, 6.1%, 10.8%, 11.2% and 13.0% ΔmtDNA4696, respectively. The results are shown in Figure 2b.

### (Result)

Four F1 mice had 27.4%, 38.0%, 47.0% and 48.7% ΔmtDNA4696, respectively. Seven F2 mice had 19.6%, 39.1%, 41.3%, 63.9%, 73.2%, 73.5% and 74.8% ΔmtDNA4696.

These results suggest that ΔmtDNA4696 is transmitted from F0 mother mice through the female germ line to their progenies. In addition, the reason why no mice having 90% or more ΔmtDNA4696 in the muscle could be produced is because percentages higher than 90%. were lethal to the mice.

Mice possessing higher proportions of ΔmtDNA4696 could be obtained through successive breedings.

### (4) Comparison of the amounts of ΔmtDNA4696 within various types of tissues from a mouse

Next, the proportions of ΔmtDNA4696 within various types of tissues from a mouse were compared using 5 male mice [two F1 mice (5-weeks-old), one F2 mouse (17-weeks-old), and two F2 mice (21-weeks-old)]. 10 types of tissues were compared: brain, lung, heart, liver, spleen, pancreas, kidney, testis, skeletal muscle and blood. Figure 2c histogram shows the result.

### (Result)

No significant difference was observed in the distribution of ΔmtDNA4696 among tissues within each mouse.

### (5) Correlation between the proportion of ΔmtDNA4696 and COX activity in a single muscle fiber

Five male mice (21-weeks-old) having 47.3%, 70.0%, 79.0%, 82.8% and 84.8% ΔmtDNA4696 were selected by muscle biopsy from F2 mice. Two serial cryosections (10 µm and 20 µm in thickness) were prepared from the skeletal muscle (musculus soleus) of each mouse. These sections were used to examine whether there is a correlation between a reduction in COX activity and the proportion of ΔmtDNA4696.

First, COX staining was performed by incubating the 10 µm section prepared from the musculus soleus of the mice carrying 84.8% ΔmtDNA4696 in a reaction solution (DAB 60mg, 0.1M acetate buffer (pH5.6) 27ml, 1% MnCl₂ (pH5.5) 3ml, 0.1% H₂O₂ 0.3ml) at 37°C for 30 min. Based on the result of staining, cytoplasms of COX-negative fibers and COX-positive fibers were excised from the 20 µm section, and then the proportion of ΔmtDNA4696 in each cytoplasm was determined by quantitative PCR analysis using the following 3 primers:

Primer 1: 5'-ACCAGGGTTATTCTATGGCC-3' (SEQ ID NO: 5 corresponding to the
nucleotides 7576-7595 of normal mouse mtDNA)

Primer 2: 5'-CCGCATCGGAGACATCGGATT-3' (SEQ ID NO: 6 corresponding to the
nucleotides 12260-12280 of normal mouse mtDNA)

Primer 3: 5'-GTGTAGTAGTGCTGAAACTGG-3' (SEQ ID NO: 7 corresponding to the
nucleotides 12479-12459 of normal mouse mtDNA, labeled with FAM)

PCR conditions consist of 30 cycles of 94 °C for 30 sec, 55 °C for 30 sec, and 72 °C for 1 min for 1^{st} PCR and 10 cycles of 94 °C for 1 min, 48 °C for 1 min, and 72 °C for 3 min for 2^{nd} PCR.

Mutant mtDNA (ΔmtDNA4696) and normal mtDNA were respectively detected as 210 bp and 220 bp fragments. Next, the proportions of mutant and normal mtDNA fragments contained in each fiber were quantified using the FM-BIO image analyzer (HITACHI).

Figure 3 shows the observed histological and morphological abnormalities. In addition, Fig. 3a and 3c show findings obtained from normal 21-weeks-old male mice (ICR, control).

### (Result)

No COX-negative fiber was observed in the mouse skeletal muscle samples possessing 47.3% and 70.0% ΔmtDNA4696. However, in the mouse skeletal muscle samples possessing 79.0%, 82.8% and 84.8% ΔmtDNA4696, COX-positive fibers (that is, stainable for COX) and COX-negative fibers (that is, unstainable for COX) were distributed in mosaic. Figure 3b shows the findings obtained from the skeletal muscle of mice possessing 84.8% ΔmtDNA4696. In Fig. 3b, fiber samples numbered 1 to 7 possessed 95.6%, 90.0%, 86.5%, 81.9%, 82.2%, 83.2%, and 77.3% ΔmtDNA4696, respectively. COX-positive fiber samples (1, 2 and 3) possessed 90.7 ± 3.8 % ΔmtDNA4696 on average, while COX-negative fiber samples (4, 5, 6 and 7) possessed 81.2 ± 2.3 % on average.

Further, in mice observed to have COX-negative fibers in the skeletal muscle, cells having COX activity and cells lacking COX activity were distributed also in mosaic in the cardiac muscle tissue. Figure 3d shows the findings obtained for the mouse cardiac muscle possessing 84.8% ΔmtDNA4696.

These results suggest that there is a good correlation between a reduction in COX activity and the proportion of ΔmtDNA4696. Specifically, all the muscle fibers possessing more than 85% ΔmtDNA4696 lacked COX activity, and the muscle fibers possessing less than 85% ΔmtDNA4696 had COX activity.

Furthermore, most F1 to F2 mice possessing a higher proportion of ΔmtDNA4696 died as a result of weakness. Common macroscopic findings on such mice were ischemia and enlarged kidney with granulated surface. As shown in Fig. 3e, a 38-weeks-old F1 male mouse was shown to have severe ischemia based on its pale ears and tail. This mouse had 58.7% ΔmtDNA4696 in the muscle biopsy sample collected at 7-weeks-old, however, died of kidney failure on the next day of photo shooting.

Figure 3f shows the kidney derived from the above mouse (right) and from a normal male mouse (left) at the same age (weeks-old). The mouse kidney possessing ΔmtDNA exhibited a granular surface (Fig. 3f, right) and possessed 88.2% ΔmtDNA4696. Other F1 to F3 mice which died of kidney failtures at 21- to 38-weeks-old were shown to have large amounts of ΔmtDNA4696 (64.0 ± 10.4 %, n=5) in muscle biopsy samples. Renal tissues of these mice possessed 80% or more (85.4 ± 5.4 %, n=5) ΔmtDNA4696 upon death.

Figure 3h shows the findings on the renal tissue of a 24-weeks-old male mouse having 94.0% ΔmtDNA4696; and Fig. 3g shows the kidney of a normal male mouse at the same age (week-old). The mouse renal tissue possessing ΔmtDNA was observed to have significant cortical dilation of proximal and distal renal tubules, and partially contain a substance stainable with eosin.

### (6) Effect of ΔmtDNA4696 on mouse blood component

Peripheral blood was collected from tail of 12 male mice (12 weeks-old) having ΔmtDNA4696 to measure blood pyruvate levels (mg/dL) and lactate levels (mg/dL) before and after oral administration of glucose (1.5 g/kg body weight). Thus, a correlation between changes in the levels and the proportion of ΔmtDNA4696 were examined. Blood pyruvate levels and lactate levels were measured based on changes in concentration between NAD and NADH. The results are shown in Fig. 4a and 4b.

Similarly, peripheral blood was collected from five F2 male mice (21-weeks-old) possessing 47.3%, 70.0%, 79.0%, 82.8% and 84.8% ΔmtDNA4696 same as those in (5), and then blood urea nitrogen levels and creatinine levels were measured using DryChem (Fuji Film). These levels were compared with the levels obtained for the peripheral blood of mice having normal mtDNA. The results are shown in Figure 4c.

### (Result)

As shown in Fig. 4b, mice having the greater proportions of ΔmtDNA4696 in the muscle tissue showed more increased peripheral blood lactate levels (In the figure, a diamond symbol (white) represents levels measured before glucose administration, and a diamond symbol (black) represents levels measured after the administration).

These results and findings (Fig. 3d) reported in (5) for the cardiac muscle possessing ΔmtDNA4696 revealed that a mouse having ΔmtDNA4696 can be used as a pathological model for mitochondrial disease.

As shown in Fig. 4c, mice having a high proportion of ΔmtDNA4696 showed significantly high blood urea nitrogen and creatinine levels, compared to mice having normal mtDNA.

Based on these results and findings (Fig. 3f and 3h) reported in (5) for the renal tissue possessing ΔmtDNA4696, it is assumed that the cause of death of mice having a high proportion of ΔmtDNA4696 is renal tuburogenic dysfunction.

Though renal dysfunction is not a common symptom of mitochondrial diseases, mitochondrial dysfunction in kidney has been reported. Accordingly, we hereby propose that some renal diseases with unknown pathogenesis are caused by the accumulation of mutant mtDNA.

The reason why muscle fibers possessing 85% or less ΔmtDNA4696 exhibited normal mitochondrial respiratory functions can be described well by the presence of an interaction between mitochondria possessing normal mtDNA and mitochondria possessing ΔmtDNA4696 introduced therein in a fertilized egg which is a recipient of ΔmtDNA4696. Specifically, the interaction supports the idea that we have previously proposed that mammalian mitochondria are functionally simple, and they can exchange their content as a result of their interaction.

Moreover, we have also previously reported that mtDNA of a sperm introduced in an egg cell upon fertilization is completely eliminated before the 2-cell stage. However, in the study of the present invention, we found that mitochondria of somatic cells possessing ΔmtDNA4696 can evade elimination from a fertilized egg after their introduction.

Therefore, we have succeeded in establishing mice having a large amount of ΔmtDNA4696 introduced following only two generations because of the two reasons below: that somatic cell-derived mitochondria possessing ΔmtDNA4696 introduced into embryos can evade elimination from the embryos, and that ΔmtDNA4696 has an advantage in replicating more easily than normal mtDNA, thus ΔmtDNA4696 is transmitted more predominantly to the next generation compared to normal mtDNA.

To date, though transmission of ΔmtDNA through the female germ line to the next generation has been reported for *Drosophila*, it has not been reported for human Kearns-Sayre syndrome which pathogenesis is deletion mutations. However, maternal inheritance of point mutation of mtDNA has been observed in patients suffered from other mitochondrial diseases. Induction of reduced respiratory activity requires the accumulation of 70% deletion mutant mtDNA, whereas the accumulation of 90% or more point mutant mtDNA having this pathogenicity is required to cause the reduced respiratory activity.

Hence, the toxicity of the mouse ΔmtDNA4696 provided by the present invention is less than that of human mtDNA with deletion mutation, but is almost the same as that of human mtDNA with point mutation.

In addition, though ΔmtDNA is not accumulated in human mitotic tissue, a large amount of the mouse ΔmtDNA4696 was accumulated in this tissue (Fig. 2c). The result suggests that mouse mitotic cells survive even with ΔmtDNA4696 accumulated therein.

Therefore, differences found in transmission to progeny and distribution over various tissue types between the ΔmtDNA in the mouse of the present invention and human ΔmtDNA are due to either of the following facts:
The ΔmtDNA4696 in the mouse of the present invention has a toxicity weaker than that of human ΔmtDNA; or
mouse cells are resistant against energy loss, and can survive even when mouse egg cells, embryos and mitotic tissue contain a large amount of ΔmtDNA4696, thereby enabling the maternal inheritance of the mouse ΔmtDNA4696 and the accumulation of ΔmtDNA4696 in large amounts in mitotic tissues.

It has often been reported up until now, that mitochondrial dysfunction is induced in mice by knocking out the gene, which is a factor encoded by nuclear DNA and is involved in mitochondrial functions. However, these mice cannot be pathological models of diseases caused by mutations in mtDNA.

Recently, chimera mice having chloramphenicol-resistant mtDNA in very small amount have been reported. However, such chimera mice are generated by transferring female ES cells carrying a large amount of chloramphenicol-resistant mtDNA into normal blastocyst stage embryos. In other words, such chimera mice cannot be selected using chloramphenicol, because the proportion of chloramphenicol-resistant mtDNA would sharply decrease as the mtDNAs were not transmitted through germ cells to the succeeding generations.

All references cited herein are incorporated into this specification by reference in their entirety.

### Industrial Applicability

The present invention enables to provide an effective method for introducing mutant mtDNA into cells. The present invention also enables the production of mtDNA-knockout mice carrying ΔmtDNA4696 by using mutant mtDNA in the form of ΔmtDNA4696. Since this model mouse is useful in studying in detail the transmission of ΔmtDNA and the expression mechanism in various types of tissue, it can be used as a pathological model for mitochondrial diseases. The model mouse is also applicable to verify the hypothesis that the accumulation of mutant mtDNA may be responsible for aging and age-related disorders. We are planning to pursue this study after the mice grow older, because many mitochondrial diseases develop with age. Moreover, the present invention can provide specimens at any age and at any stage of medically progressive conditions from any type of tissue possessing ΔmtDNA at various proportions. This may prove that mutations in mtDNA cause cryptogenic diseases. Finally, the present invention is also applicable to screening for a therapeutic agent and establishing gene therapies that are possible only with the model animal.

SEQUENCE LISTING

<110> Institute of Tsukuba Liaison Co., Ltd.

<120> Method for introducing mutant mitochondrial DNA into germ cell

<130> PH-1113-PCT

<140> PCT/JP00/09111
<141> 2000-12-21

<150> JP2000/9194
<151> 2000-01-18

<160> 7

<170> PatentIn Ver. 2.0

<210> 1
<211> 25
<212> DNA
<213> Artificial Sequence

<220>
<223> Description of Artificial Sequence:a forward primer:this sequence
corresponds to nucleotides 6871-6895 of normal mitochondrial DNA.

<400> 1
   aagaaaggaa ggaatcgaac cccct                                    25

<210> 2
<211> 25
<212> DNA
<213> Artificial Sequence

<220>
<223> Description of Artificial Sequence: a reverse primer:this sequence
corresponds to nucleotides 13666-13642 of normal mitochondrial DNA.

<400> 2
tatgttggaa ggagggattg gggta                                          25

<210> 3
<211> 24
<212> DNA
<213> Artificial Sequence

<220>
<223> Description of Artificial Sequence: a forward primer:this sequence
corresponds to nucleotides 7558-7581 of normal mitochondrial DNA.

<400> 3
aacagtaaca tcaaaccgac cagg                                           24

<210> 4
<211> 21
<212> DNA
<213> Artificial Sequence

<220>

<223> Description of Artificial Sequence: a reverse primer:this sequence

corresponds to nucleotides 12678-12658 of normal mitochondrial DNA.

<400> 4

ctattatcag gcctagttgg c                                                21

<210> 5

<211> 20

<212> DNA

<213> Artificial Sequence

<220>

<223> Description of Artificial Sequence: primer 1 :this sequence

corresponds to nucleotides 7576-7595 of normal mitochondrial DNA.

<400> 5

accagggtta ttctatggcc                                                  20

<210> 6

<211> 21

<212> DNA

<213> Artificial Sequence

<220>

<223> Description of Artificial Sequence: primer 2 :this sequence

corresponds to nucleotides 12260-12280 of normal mitochondrial DNA.

<400> 6

ccgcatcgga gacatcggat t                                                21

<210> 7

<211> 21

<212> DNA

<213> Artificial Sequence

<220>

<223> Description of Artificial Sequence: primer 3 :this sequence

corresponds to nucleotides 12479-12459 of normal mitochondrial DNA.

<400> 7

gtgtagtagt gctgaaactg g                                                21

## Claims

1. A method for introducing mutant mitochondrial DNA into a germ cell, which comprises fusing an enucleated cell possessing mutant mitochondrial DNA with a mitochondrial DNA-less cell to produce a cybrid, enucleating the cybrid, and fusing the cybrid with a germ cell.

2. The method of claim 1, wherein the mutant mitochondrial DNA is a deletion mutant mitochondrial DNA4696.

3. A mitochondrial DNA-knockout mouse, which is produced by transferring a germ cell produced by the method of claim 2 into a pseudopregnant mouse.

4. The mitochondrial DNA-knockout mouse of claim 3, wherein the germ cell is a pro-nuclear stage embryo.
